# EUROPEAN PATENT APPLICATION

(11) **EP 3 623 476 A1**
(43) Date of publication of application: **18.03.2020**
(21) Application number: 18797914.1
(22) Date of filing: 07.05.2018
(51) Int. Cl.: C12N 15/64, A01K 67/027, A61K 35/76, A61K 48/00, A61P 15/08, C12N 5/10, C12N 5/076

(54) **METHOD FOR INTRODUCING POLYNUCLEOTIDE TO MALE GERM CELL OR SERTOLI CELL**

(30) Priority: 08.05.2017 JP 2017092384
(71) Applicant: Kyoto University, Kyoto-shi, Kyoto 606-8501 (JP)
(72) Inventor: SHINOHARA, Takashi, Kyoto-shi Kyoto 606-8501 (JP); WATANABE, Satoshi, Kyoto-shi Kyoto 606-8501 (JP)
(74) Representative: Hoffmann Eitle
(86) International application number: PCT/JP2018/017650
(87) International publication number: WO 2018/207736

(57) **Abstract**

The disclosure includes a method of introducing a polynucleotide into a male germ cell or a Sertoli cell, comprising injecting an adeno-associated virus vector comprising the polynucleotide into the testis of a vertebrate.

## Description

### TECHNICAL FIELD

The present application claims the priority to Japanese Patent Application No. 2017-092384, the entirety of which is herein incorporated by reference.

The present application relates to the field of genetic modification and gene therapy, and in particular, includes a method of introducing a polynucleotide into a male germ cell or a Sertoli cell, and a method of producing a genetically modified animal.

### BACKGROUND

Spermatogonial stem cells are the only male germ cells with self-renewal capacity; and transduction of the cells may greatly improve the efficiency of production of genetically modified animals. Seminiferous tubules are the place of spermatogenesis and divided into the basal and adluminal compartments by the blood-testis barrier formed with Sertoli cells. Undifferentiated male germ cells including spermatogonial stem cells reside and are protected in the basal compartment. Gene transfer into spermatogonial stem cells using retrovirus vectors have been reported. In order for the vectors to reach spermatogonial stem cells, however, the vectors have to be injected into immature seminiferous tubules in which the blood-testis barrier is not formed. Microinjection into immature seminiferous tubules requires a high-skill as they are extremely thin, and has to be performed in a limited period of time.

Sertoli cells support spermatogenesis and are suggested to be involved in male infertility. Gene transfer into Sertoli cells by microinjection of lentivirus or adenovirus vectors to seminiferous tubules has been reported. It is concerned, however, that lentiviruses can mutate germ cells by insertion of a transgene and adenovirus vectors can induce inflammatory responses.

### CITATION LIST

### PATENT DOCUMENTS

Patent Document 1: WO2005/115133

### NON PATENT DOCUMENTS

Non Patent Document 1: Ikawa M, Tergaonkar V, Ogura A, Ogonuki N, Inoue K, Verma IM. Restoration of spermatogenesis by lentiviral gene transfer: offspring from infertile mice. Proc Natl Acad Sci USA 2002; 99: 7524-7529.
Non Patent Document 2: Kanatsu-Shinohara M, Ogura A, Ikegawa M, Inoue K, Ogonuki N, Tashiro K, Toyokuni S, Honjo T, Shinohara T. Adenovirus-mediated gene delivery and in vitro microinsemination produce offspring from infertile male mice. Proc Natl Acad Sci USA 2002; 99: 1383-1388.
Non Patent Document 3: Kanatsu-Shinohara M, Ogonuki N, Inoue K, Miki H, Ogura A, Toyokuni S, Shinohara T. Long-term proliferation in culture and germline transmission of mouse male germline stem cells. Biol Reprod 2003; 69: 612-616.
Non Patent Document 4: Kanatsu-Shinohara M, Toyokuni S, Shinohara T. Transgenic mice produced by retroviral transduction of male germ line stem cells in vivo. Biol Reprod 2004;71:1202-1207.

### SUMMARY

### PROBLEM TO BE SOLVED

An object of the disclosure is to provide an improved method for introducing a polynucleotide into a male germ cell or a Sertoli cell and its application.

### SOLUTION TO PROBLEM

In one aspect, the invention provides a method of introducing a polynucleotide into a male germ cell or a Sertoli cell, comprising injecting an adeno-associated virus vector comprising the polynucleotide into the testis of a vertebrate.

In another aspect, the invention provides a method of producing a vertebrate comprising a male germ cell or a Sertoli cell into which a polynucleotide is introduced, comprising injecting an adeno-associated virus vector comprising the polynucleotide into the testis of a vertebrate.

In another aspect, the invention provides a method of producing a genetically modified vertebrate, comprising
injecting an adeno-associated virus vector into the testis of a vertebrate to form a genetically modified sperm, and
fertilizing an egg with the genetically modified sperm to obtain a genetically modified individual.

In another aspect, the invention provides a method of producing a genetically modified sperm, comprising injecting an adeno-associated virus vector into the testis of a vertebrate to form a genetically modified sperm.

In another aspect, the invention provides a composition comprising an adeno-associated virus vector for introducing a polynucleotide into a male germ cell or a Sertoli cell in the testis of a vertebrate.

### EFFECTS OF INVENTION

According to the present invention, a polynucleotide is readily introduced into a male germ cell or a Sertoli cell. Adeno-associated virus (herein also referred to as AAV) vectors are safe and easy to handle and have already been used in gene therapy, and thus would be useful in a wide variety of applications.

### DESCRIPTION OF DRAWINGS

Fig. 1A shows transduction of R26R-EYFP mouse testes by an adenovirus or lentivirus vector (A). A scheme showing the routes of microinjection. Using glass capillary, the virus particles were microinjected into the seminiferous tubules via the efferent ducts for tubular injection, or into the interstitial region of the testis for interstitial injection. Interstitial cells are found adjacent to the seminiferous tubules.
Fig. 1B shows transduction of R26R-EYFP mouse testes by an adenovirus or lentivirus vector (B). Macroscopic appearance of R26R-EYFP mouse testes 7 days (1 weeks) after microinjection of AxCANCre and CSII-EF1-Cre.
Fig. 1C shows transduction of R26R-EYFP mouse testes by an adenovirus or lentivirus vector (C). Immunostaining of R26R-EYFP mouse testes for undifferentiated spermatogonia (GFRA1), Sertoli (VIM), peritubular (ACTA2), and Leydig (STAR) cell markers 7 days after microinjection.
Fig. 2A shows screening of AAV serotypes (A). Macroscopic appearance of wild-type mouse testes 7 days after microinjection of mCherry-expressing AAV. The upper photographs show the results of tubular injection 1 week postinjection, and the lower photographs show the results of interstitial injection 1 week postinjection.
Fig. 2B shows screening of AAV serotypes (B). Immunostaining of AAV1-, AAV8-, or AAV9-mCherry-injected testes for undifferentiated spermatogonia (GFRA1) and Sertoli cell (WT1) markers 7 days after microinjection of AAV-mCherry.
Fig. 2C shows screening of AAV serotypes (C). Quantification of immunostaining. At least 3 tubules were counted for each of tubular and interstitial injection. The ratio of mCherry⁺ cells in WT1⁺ Sertoli cells (%) (mCherry⁺ cells/WT1⁺ Sertoli cells (%)), and that of mCherry⁺ cells in marker-positive cells (%) (mCherry⁺ cells/marker⁺ cells) (%)) are shown.
Fig. 2D shows screening of AAV serotypes (D). Immunostaining of AAV7M8-mCherry-injected testes for an undifferentiated spermatogonia (GFRA1) marker 7 days after microinjection of AAV-mCherry.
Fig. 3A shows kinetics of transduction analyzed by microinjection of AAV9-Cre into R26R-EYFP mice (A). Macroscopic appearance of R26R-EYFP mouse testes at indicated time points after AAV9-Cre microinjection.
Fig. 3B shows kinetics of transduction analyzed by microinjection of AAV9-Cre into R26R-EYFP mice (B). Immunostaining of R26R-EYFP mouse testes for undifferentiated spermatogonia (GFRA1) and Sertoli cell (WT1) markers.
Fig. 3C shows real-time PCR analysis of *Gdnf, Fgf2,* and *Kitl* expression 2 days after microinjection of AAV1-mCherry or AAV9-mCherry into wild-type mice (n = 3). Relative expression levels are shown. The columns show the results of control (vehicle), AAV1-mCherry, and AAV9-mCherry, respectively, from the left.
Fig. 3D shows immunostaining of CLDN11 7 days after microinjection of vehicle, AAV1-mCherry or AAV9-mCherry.
Fig. 3E shows functional analysis of the blood-testis barrier (BTB). Three days after tubular or interstitial injection of AAV8-mCherry, AAV1-mCherry or AAV9-mCherry to wild-type or *Cldn11* knockout mice, biotin was microinjected to the testis interstitium. The samples were collected after 30 minutes of microinjection.
Fig. 4A shows functional analysis of spermatogonial stem cell (SSC) transduction by AAVs (A). The scheme shows dissociation of cells from AAV1- or AAV9-Cre injected testis (tubular or interstitial), microinjection into the testes of busulfan-treated mice, colony count, microinsemination, genotyping of offspring, and detection of AAV transgene.
Fig. 4B shows functional analysis of SSC transduction by AAVs (B). Macroscopic appearance of R26R-EYFP mouse testes at the time of donor cell collection.
Fig. 4C shows functional analysis of SSC transduction by AAVs (C). Macroscopic appearance of recipient testes.
Fig. 4D shows functional analysis of SSC transduction by AAVs (D). Staining of recipient testes for spermatogenesis markers (peanut agglutinin, PNA) showing normal appearance of donor-derived spermatogenesis.
Fig. 4E shows functional analysis of SSC transduction by AAVs (E). Colony counts (n = 18 to 19) are shown as colonies/10⁵ cells.
Fig. 4F shows functional analysis of SSC transduction by AAVs (F). Offspring born after microinsemination using AAV9-infected R26R-EYFP donor testis cells.
Fig. 4G shows functional analysis of SSC transduction by AAVs (G). EYFP fluorescences in the offspring. EYFP fluorescences in testis, kidney, liver, and brain are shown with the bright field observation for offspring from wild-type mice or mice that received tubular or interstitial injection.
Fig. 4H shows functional analysis of SSC transduction by AAVs (H). PCR analysis of Cre-mediated deletion. Tail DNA samples from offspring produced after tubular or interstitial injection were analyzed using indicated primers.
Fig. 5A shows improvement of AAV transduction (A). Macroscopic appearance of wild-type testes that received AAV9-2YF-mCherry.
Fig. 5B shows improvement of AAV transduction (B) . Macroscopic appearance of wild-type testes that received AAV9-mCherry with neuraminidase.
Fig. 5C shows improvement of AAV transduction (C) . Immunostaining of wild-type testes for undifferentiated spermatogonia (GFRA1) and Sertoli cell (WT1) markers.
Fig. 5D shows improvement of AAV transduction (D). Quantification of immunostaining. At least 3 tubules were counted for each of tubular and interstitial injection. The ratio of WT1⁺ cells in mCherry⁺ cells (%) (WT1⁺ cells/mCherry⁺ cells (%)), and that of GFRA1⁺ cells in mCherry⁺ cells (%) (GFRA1⁺ cells/mCherry⁺ cells) (%)) are shown. The left and right columns show the results with vehicle and neuraminidase, respectively.
Fig. 6A shows treatment of infertility in *Kitl^{sl}*/*Kitl*^{*sl*- *d*} mice by AAV9-Kitl infection (A). Macroscopic, histological and lectin-histochemical appearance of *Kitl^{sl}*/*Kitl*^{*sl*-*d*} mouse testes after microinjection of AAV9-Kitl.
Fig. 6B shows treatment of infertility in *Kitl^{sl}*/*Kitl*^{*sl*- *d*} mice by AAV9-Kitl infection (B). Offspring born after microinsemination using sperms generated after tubular injection of Kitl-expressing AAV9.
Fig. 6C shows treatment of infertility in *Kitl^{sl}*/*Kitl*^{*sl*- *d*} mice by AAV9-Kitl infection (C). Offspring born after microinsemination using sperms generated after interstitial injection of AAV9-Kitl.
Fig. 6D shows treatment of infertility in *Kitl^{sl}*/*Kitl*^{*sl*- *d*} mice by AAV9-Kitl infection (D). Southern blot analysis of *EcoR I*-digested DNA using a Kitl cDNA probe. DNA samples were collected from representative offspring born after microinsemination using sperm generated after tubular injection of AAV9-Kitl. The wild-type locus produced hybridization bands at 4.3 and 13 kb, the *Kitl*^{*sl*-*d*} locus produced bands at 4.3 and 7 kb, and the *Kitl^{sl}* locus produced no bands (same in Fig. 6E).
Fig. 6E shows treatment of infertility in *Kitl^{sl}*/*Kitl^{sl- d}* mice by AAV9-Kitl infection (E). Southern blot analysis of *EcoR I*-digested DNA using a Kitl cDNA probe. DNA samples were collected from representative offspring born after microinsemination using sperm generated after interstitial injection of AAV9-Kitl.
Fig. 7A shows analysis of AAV9 integration in the offspring (A). Tail DNA samples from offspring produced after tubular or interstitial injection of AAV9-Cre were analyzed using Cre-specific primers.
Fig. 7B shows analysis of AAV9 integration in the offspring (B). Tail DNA samples from offspring produced after tubular or interstitial injection of AAV9-Kitl were analyzed using AAV9-specific primers.
Fig. 8 shows transduction of rabbit testicular cells by AAVs. mCherry: Staining with an anti-mCherry antibody. Merge: Results of staining with an anti-mCherry antibody and Hoechst 33342 are merged.
Fig. 9 shows transduction of marmoset testicular cells by AAVs. Merge: Results of staining with an anti-mCherry antibody, Hoechst 33342, and an anti-SSEA3 or anti-WT1 antibody are merged.

### DESCRIPTION OF EMBODIMENTS

Unless otherwise indicated, the terms used herein are read as commonly understood by a skilled person in the technical fields such as organic chemistry, medical science, pharmaceutical science, molecular biology, and microbiology. Several terms used herein are defined as described below. The definitions herein take precedence over general understandings.

When a numerical value is accompanied with the term "about", it is intended to represent the value ± 10% of that value. A range defined with values of the lower and upper limits covers all values between the lower and upper limits, including the values of the both limits. When a range is accompanied with the term "about", the both limits are read as accompanied with the term. For example, "about 20 to 30" is read as "20±10% to 30±10%".

Examples of vertebrates include mammals, birds, fishes, amphibians and reptiles. Mammals include, but are not limited to, for example, mice, rats, hamsters, guinea pigs, rabbits, pigs, cattle, goats, horses, sheep, minks, dogs, cats, monkeys, rhesus monkeys, marmosets, orangutans, chimpanzees, and humans. Birds include chickens, quails, ducks, geese, turkeys, ostriches, emus, camel birds, guinea fowls, and pigeons. In a preferred embodiment, the vertebrate is a mammal. In an embodiment, the mammal is a rodent, lagomorpha or primate. In a further embodiment, the mammal is a mouse, rat, hamster, guinea pig, rabbit, monkey, rhesus monkey, marmoset, orangutan, chimpanzee, or human. In a further embodiment, the mammal is a human.

The vertebrate may be an animal in any developmental stage In an embodiment, the vertebrate is an animal having the blood-testis barrier (herein also referred to as BTB). The animal having the blood-testis barrier can be an animal in a developmental stage after the blood-testis barrier is formed. When the blood-testis barrier is formed varies depending on the types of vertebrates, and for example, 2-week old in mice, 2- to 3-week old in rats, 10-week old in rabbits, 20- to 32-week old in cattle, 10- to 15- month old in rhesus monkeys, 5- to 6-month old in marmosets. In an embodiment, the animal having the blood-testis barrier is an adult animal. In a different embodiment, the vertebrate is an animal not having the blood-testis barrier. The animal not having the blood-testis barrier may be, for example, an animal in a developmental stage before the blood-testis barrier is formed, which is herein referred to as "an juvenile animal". In a preferred embodiment, the vertebrate is an animal having the blood-testis barrier.

The adeno-associated virus (AAV) vector of the disclosure may be any AAV vector that is directional to male germ cells (preferably, sperm stem cells) or Sertoli cells and can pass through the basement membrane or blood-testis barrier of seminiferous tubules. The AAV vector may have a natural or an artificially-modified capsid protein. Preferably, the AAV vector is capable of passing through the basement membrane of seminiferous tubules. A suitable AAV vector can be selected by examining whether the vector infects cells of interest when injected into the testis of a vertebrate according to the description in the examples. In a preferred embodiment, the AAV vector is AAV1, AAV9, or AAV7M8, more preferably AAV1 or AAV9, or a variant thereof that retains its directionality and ability to pass through the basement membrane or blood-testis barrier. How to obtain a desired variant is known in the art. For example, a desired variant may be obtained by modifying a capsid protein and examining the property of the resulting vector according to the description in the examples. A desired variant may also be obtained by preparing a library of AAVs having capsid proteins mutated by a technique such as DNA shuffling or error-prone PCR and screening the library.

A polynucleotide comprised in an AAV vector may be, but not limited to, a polynucleotide encoding a protein or peptide, or a polynucleotide encoding a nucleic acid molecule such as an antisense nucleic acid, siRNA, miRNA, stRNA, ribozyme, or decoy nucleic acid. In an embodiment, the polynucleotide is a polynucleotide encoding a fluorescent protein such as GFP, eGFP, BFP, YFP, EYFP, CFP, RFP, dsRed or mCherry (herein also referred to as a marker gene).

The polynucleotide may be a polynucleotide encoding a protein or nucleic acid molecule for genome editing with CRISPR/Cas9, TALEN or ZFN. AAV vectors can modify the genome of a male germ cell or Sertoli cell when combined with genome editing technology, although they are not normally inserted into the genome of a cell. The protein or nucleic acid molecule for genome editing with CRISPR/Cas9 may be, for example, Cas9 protein, which is an endonuclease, or a guide RNA (gRNA), which recruits the Cas9 protein to a target sequence, or a donor vector to be introduced into a double stranded cleavage site in the genome.

The polynucleotide may comprise a regulatory element such as a promoter or enhancer. The promoter may be any promoter as long as it can regulate the expression of the polynucleotide in a cell. The promoter may be, for example, CAG promoter, SRα promoter, EF1α promoter, CMV promoter, PGK promoter, U6 promoter, a tissue non-specific promoter such as tRNA promoter, or a tissue specific promoter such as liver-specific α1AT promoter, skeletal muscle-specific α-actin promoter, neuron-specific enolase promoter, or vascular endothelial cell-specific tie promoter. The promoter can be appropriately selected depending on the intended purpose.

The size of the polynucleotide comprised in an AAV vector is typically, but not limited to, up to about 4.7 kbp. An AAV vector may comprise two or more polynucleotides, or two or more AAV vectors may be injected in combination.

The AAV vector may be produced by any method known in the art. For example, the AAV vector may be produced by transfecting packaging cells such as HEK293 cells or its variant AAV-293 cells with, 1) an AAV vector plasmid, which has inverted terminal repeats (ITRs) of AAVs at both ends and a polynucleotide of interest inserted between the ITRs, 2) an AAV helper plasmid, which has the Rep gene and the Cap gene that are required for AAV replication or particle formation, and 3) an adenovirus helper plasmid, which has a helper gene of adenoviruses that is required for the growth of AAVs. Production of the AAV vector according to such a process can use a commercially available kit such as AAV Helper-Free System (Agilent Technologies).

The AAV vector may be injected into the testis with a reagent for increasing the transduction efficiency. Examples of such reagents include neuraminidase, a proteasome inhibitor such as MG132, Eeyarestatin I, tritiated thymidine, cisplatin, etoposide, a calpain inhibitor, or a ubiquitin ligase inhibitor.

In the present disclosure, the AAV vector is injected into the testis of a vertebrate *in vivo.* The AAV vector may be injected into any site of the testis. The injection site can be the testis interstitium, seminiferous tubules, efferent ducts, or rete testis. In an embodiment, the AAV vector is injected into the testis interstitium.

The AAV vector of the disclosure passes through the basement membrane or blood-testis barrier of seminiferous tubules and infects a male germ cell or Sertoli cell that is present in the basal compartment. Thus, the AAV vector of the disclosure is not necessary to be injected into the seminiferous tubules, efferent ducts, or rete testis of an animal not having the blood-testis barrier, and may be injected into the testis at any time and any site. Further, the AAV vector does not require a process of reducing testicular cells as described in WO99/038991, and the method of the disclosure does not contain such a process.

The amount of the AAV vector to be injected into a subject varies depending on the subject, but may be, for example, about 1 × 10¹⁰ to 1 × 10¹¹ viral particles per 20 g body weight.

As used herein, a male germ cell can be a germ cell at any developmental stage. The male germ cell may be a spermatogonial stem cell (herein also referred to as SSC), spermatogonium, spermatocyte (primary or secondary), spermatid, or sperm. In an embodiment, the male germ cell is a spermatogonial stem cell.

In an embodiment, a genetically modified sperm can be obtained by allowing a male germ cell into which a polynucleotide has been introduced to form a sperm. Spermatogonial stem cells have self-renewal capacity and thus genetically modified sperms can be produced continuously from genetically modified spermatogonial stem cells. On the other hand, germ cells other than the spermatogonial stem cells do not have self-renewal capacity and sperms produced from any of these germ cells are considered to disappear after a period required for one cycle of spermatogenesis (approximately 35 days in mice). Therefore, when a genetically modified sperm exists after that period from the injection of an AAV vector, it is determined that a polynucleotide has been introduced into a spermatogonial stem cell. A genetically modified sperm can be confirmed or selected by a known method. For example, when a marker gene is introduced (along with a different polynucleotide, or alone), a genetically modified sperm can be confirmed or selected based on the expression of the marker gene.

The genetically modified sperm thus obtained may be used to obtain a genetically modified animal by fertilizing an egg to develop an individual animal. An egg as used herein means a fertile female gamete which a sperm can fertilize, such as an egg cell or oocyte. Typically, an egg and a sperm are from the same species of vertebrates. Fertilization of an egg with a sperm can be carried out by a known method such as natural mating or artificial insemination by a technique such as microinsemination (ICSI) or *in vitro* fertilization (IVF). For example, a male into which an AAV vector has been injected may be mated with a female. It is not essential to confirm before the mating that the male into which an AAV vector has been injected has a genetically modified sperm. Alternatively, a genetically modified animal may be obtained by fertilizing an egg by artificial insemination with a sperm obtained from an animal into which an AAV vector has been injected and returning the fertilized egg to the uterus of a pseudopregnant animal.

Whether a genetically modified animal is obtained can be confirmed by a known method. For example, it may be confirmed by examining the expression of a marker gene, by collecting the genomic DNA and subjecting the same to PCR or Southern blotting, or by examining the presence of an expression product (e.g., a protein) from the introduced polynucleotide.

A disease caused by genetic abnormality of a male germ cell or a Sertoli cell can be treated by introducing a polynucleotide into the cell. For example, Sertoli cells have been suggested to be involved in male infertility, and thus genetic modification of a Sertoli cell can treat such a disease. The polynucleotide may encode a protein, peptide or nucleic acid molecule that compensates the function of a protein decreased or lost, or suppresses the function of a protein enhanced, by the genetic abnormality.

A composition comprising an AAV vector may comprise a pharmaceutically acceptable carrier and/or additive in addition to the AAV vector as an active ingredient. The pharmaceutically acceptable carrier may be physiological saline or any other physiologically acceptable buffer solution. The additive may be a solubilizing agent, pH adjusting agent, preservative, or stabilizing agent. The dosage form may be, but not limited to, an injection such as a liquid injection or a solid injection that is dissolved before use (e.g., freeze-dried injection). The composition comprising an AAV vector may be provided as a kit. The kit may further comprise an additional component, such as a buffer solution for dissolution before use, or instructions for use of the kit.

The dosage of the AAV vector is appropriately determined depending on the subject to which the AAV vector is administered, and may be, for example, about 10⁹ to 10¹⁵ vg (vector genome) , preferably 10¹⁰ to 10¹⁴ vg or 10¹⁰ to 10¹³ vg per kg body weight.

The followings are illustrative embodiments of the disclosure.
1. A method of introducing a polynucleotide into a male germ cell or a Sertoli cell, comprising injecting an adeno-associated virus vector comprising the polynucleotide into the testis of a vertebrate.
2. A method of producing a vertebrate comprising a male germ cell or a Sertoli cell into which a polynucleotide is introduced, comprising injecting an adeno-associated virus vector comprising the polynucleotide into the testis of a vertebrate.
3. The method according to item 1 or 2, wherein the adeno-associated virus vector is AAV1, AAV9 or AAV7M8, preferably AAV1 or AAV9.
4. The method according to any one of items 1-3, wherein the male germ cell is a spermatogonial stem cell.
5. The method according to any one of items 1-4, wherein the vertebrate has the blood-testis barrier.
6. The method according to any one of items 1-5, wherein the adeno-associated virus vector is injected into the testis interstitium.
7. The method according to any one of items 1-6, wherein the vertebrate is a non-human vertebrate.
8. The method according to any one of items 1-7, wherein the vertebrate is a mammal.
9. The method according to item 8, wherein the mammal is a rodent, lagomorpha or primate.
10. The method according to any one of items 1-6, wherein the vertebrate is a human.
11. A method of producing a genetically modified vertebrate, comprising
   injecting an adeno-associated virus vector into the testis of a vertebrate to form a genetically modified sperm, and
   fertilizing an egg with the genetically modified sperm to obtain a genetically modified individual.
12. The method according to item 11, wherein the adeno-associated virus vector is AAV1, AAV9 or AAV7M8, preferably AAV1 or AAV9.
13. The method according to item 11 or 12, wherein the vertebrate has the blood-testis barrier.
14. The method according to any one of items 11-13, wherein the adeno-associated virus vector is injected into the testis interstitium.
15. The method according to any one of items 11-14, wherein the vertebrate is a non-human vertebrate.
16. The method according to any one of items 11-15, wherein the vertebrate is a mammal.
17. The method according to item 16, wherein the mammal is a rodent, lagomorpha or primate.
18. A method of producing a genetically modified sperm, comprising injecting an adeno-associated virus vector into the testis of a vertebrate to form a genetically modified sperm.
19. The method according to item 18, wherein the adeno-associated virus vector is AAV1, AAV9 or AAV7M8, preferably AAV1 or AAV9.
20. The method according to item 18 or 19, wherein the vertebrate has the blood-testis barrier.
21. The method according to any one of items 18-20, wherein the adeno-associated virus vector is injected into the testis interstitium.
22. The method according to any one of items 18-21, wherein the vertebrate is a non-human vertebrate.
23. The method according to any one of items 18-22, wherein the vertebrate is a mammal.
24. The method according to item 23, wherein the mammal is a rodent, lagomorpha or primate.
25. A composition comprising an adeno-associated virus vector for introducing a polynucleotide into a male germ cell or a Sertoli cell in the testis of a vertebrate.
26. The composition according to item 25, wherein the composition is for treating a disease.
27. A composition comprising an adeno-associated virus vector for treating a disease caused by genetic abnormality of a male germ cell or a Sertoli cell.
28. The composition according to item 26 or 27, wherein the composition is for treating male infertility.
29. The composition according to any one of items 25-28, wherein the vertebrate is a human.
30. The composition according to any one of items 25-29, wherein the adeno-associated virus vector is AAV1, AAV9 or AAV7M8, preferably AAV1 or AAV9.
31. The composition according to any one of items 25-30, wherein the vertebrate has the blood-testis barrier.
32. The composition according to any one of items 25-31, wherein the composition is injected into the testis of a vertebrate.
33. The composition according to any one of items 25-32, wherein the composition is injected into the testis interstitium of a vertebrate.
34. A method of treating a disease caused by genetic abnormality of a male germ cell or a Sertoli cell, comprising injecting an adeno-associated virus vector into the testis of a vertebrate.
35. The method according to item 34, wherein the disease is male infertility.
36. The method according to item 34 or 35, wherein the vertebrate is a human.
37. The method according to any one of items 34-36, wherein the adeno-associated virus vector is AAV1, AAV9 or AAV7M8, preferably AAV1 or AAV9.
38. The method according to any one of items 34-37, wherein the vertebrate has the blood-testis barrier.
39. The method according to any one of items 34-38, wherein the adeno-associated virus vector is injected into the testis interstitium of a vertebrate.

The following examples are provided for illustrative purposes and do not limit the invention in any sense.

### Example 1

### 1. Methods

### (1) Virus production

For production of AAV vectors, an AAV vector plasmid (pAAV-CAG-mCherry or pAAV-CAG-Cre), an adenovirus helper plasmid (pHelper; Agilent Technologies, Santa Clara, CA), and an AAV helper plasmid (pAAV1, pAAV-RC [Agilent Technologies], pXR5, pAAV6, pAAV6.2, pAAV7, pAAV7M8, pAAV8, pAAV9, pAAV10, pAAV11, pAAVhu11, Anc80L65, pAAV-DJ or pAAV-DJ8) were transiently transfected into AAV-293 cells (Agilent Technologies). The virus titer was determined by real-time polymerase chain reaction (PCR) using FastStart Universal SYBR Green Master Mix (Roche, Penzberg, Germany) and specific primers. A lentivirus expressing Cre (CSII-EF-Cre-IRES2-Puro) and its production by transient 293T transfection were described in Morimoto H et al. (Biol Reprod 2015;92:147). The virus culture supernatant was concentrated by ultracentrifugation at 194,000 × g for 2 hours. The virus titer was determined by using a qPCR Lentivirus Titration Kit (Abm, BC, Canada). An adenovirus expressing Cre (AxCANCre; RIKEN BRC, Tsukuba, Japan) was produced by 293 cells and prepared using CsCl centrifugation, and the virus titer was determined as described in Takehashi et al. (Proc Natl Acad Sci USA 2007; 104: 2596-2601). The virus titers of the AAV vector, lentivirus, and adenovirus were 1 × 10¹² viral particles/ml, 3 × 10⁸ viral particles/ml, and 1.7 × 10⁹ pfu (plaque forming unit)/ml, respectively, unless otherwise indicated.

### (2) Animals and transplantation

For *in vivo* screening and tracer experiments, 4- to 5-week old C57BL/6 (B6) × DBA/2 F1 (BDF1) mice were used. *Cldn11* knockout mice (3-month old) were used as a positive control for tracer experiments. In some experiments, 4- to 8-week old transgenic Gt (ROSA) 26Sor^{tml (EYFP)Cos} mice (herein also referred to as R26R or R26R-EYFP) (Srinivas et al, BMC Dev Biol 2001;1:4) were used. For fertility restoration experiments, 4- to 5-week old *Kitl^{sl}*/*Kitl^{sl-d}* mice were used (Japan SLC, Shizuoka, Japan).

### (3) Microinjection of virus particles and spermatogonial transplantation

For tubular injection, virus particles were introduced into the seminiferous tubules via the efferent ducts. Approximately 10 and 4 µl of them were administered to the testes of BDF1 and *Kitl^{Sl}*/*Kitl^{Sl-d}* mice, respectively. For interstitium injection, the same amount of virus was microinjected into the interstitial region of the testis. In some experiments, neuramnidase (Sigma, St. Lois, MO) was used to enhance infectivity.

For spermatogonial transplantation, donor testis cells were dissociated into single cells by two-step enzymatic procedure using collagenase and trypsin (both from Sigma). The cells were microinjected into the efferent ducts of BDF1 mice that had been treated with busulfan (44 mg/kg, Sigma) at 4 weeks of age. Each injection filled 75-85% of the seminiferous tubules. All busulfan-treated recipient mice were used at 4 to 8 weeks after busulfan treatment.

### (4) Tracer experiment

Sulfo-NHS-LC-biotin (7.5 mg/ml; 557 D; Thermo Fisher Scientific) was dissolved in phosphate-buffered saline (PBS). Approximately 20 µl of the solution was microinjected into the testis interstitium. After 30 min, the animals were killed, and their testes were immediately removed and fixed in 4% paraformaldehyde at 4°C overnight. The samples were embedded in Tissue-Tek OCT compound, and processed for cryosectioning. Sulfo-NHS-LC-biotin was detected by incubation with Alexa fluor 488-conjugated streptavidin (BD Biosciences) before counterstaining.

### (5) Analysis of recipient testes

For assessment of SSC activity, recipients were sacrificed 2 months after transplantation, and donor cell colonization was examined under UV light. Germ cell clusters were defined as colonies when they occupied the entire basal surface of the tubule and were at least 0.1 mm in length. To evaluate donor cell colonization by histological analysis, sections were viewed at 400 x magnification to determine the extent of colonization in the testis, and images of the sections under an inverted microscope equipped with a CCD camera (DP70, Olympus, Tokyo, Japan) were collected using the Photoshop software (Adobe, San Jose, CA). All sections were stained with Hoechst 33342.

### (6) Immunohistochemistry

For immunohistochemistry, testis samples were fixed in 4% paraformaldehyde for 2 hours. Then the samples were embedded in Tissue-Tek OCT compound (Sakura Finetek, Tokyo, Japan) for cryosectioning. Immunostaining of cryosections was carried out by treating the samples with 0.1% Triton-X in PBS. The samples were immersed in blocking buffer (0.1% Tween 20, 1% bovine serum albumin and 1% goat serum in PBS) for more than one hour, and then incubated with the indicated primary antibodies at 4 °C overnight. After washed three times with PBS, the samples were incubated with the secondary antibody.

### (7) Analysis of gene expression

Total RNA was isolated using TRIzol® (Invitrogen, Carlsbad, CA), and single-strand cDNA was synthesized using the Verso cDNA Synthesis Kit (Thermo Fisher Scientific, Waltham, MA) and used for RT-PCR. For real-time PCR, the StepOnePlus™ Real-Time PCR system and FastStart Universal SYBR Green PCR Master Mix (Roche, Basel, Switzerland) were used according to the manufacturer's protocol (Applied Biosystems, Warrington, UK). Transcript levels were normalized relative to those of *Hprt.*

### (8) DNA analysis

Genomic DNA was isolated from the offspring by standard procedure using phenol/chloroform extraction and ethanol precipitation. Deletion of the floxed allele and virus integration was estimated by PCR.

For detection of virus DNA by Southern blotting, 20 µg of DNA was digested with *EcoR I,* and separated in a 1.0% agarose gel. DNA was transferred and blotted onto a nylon membrane (Hybond-N+; Amersham Biosciences, Buckinghamshire, UK). Hybridization was performed according to a standard protocol using a 434-bp *Nci I-Bgl* I fragment of the full-length Sl cDNA as a probe. The membrane was hybridized for 16 hours at 65°C with a ³²P-labeled probe.

### (9) Microinsemination

AAV-injected testes were refrigerated overnight and were used for microinsemination on the next day after collection. The seminiferous tubules of recipient testes were dissected, and dissociated by repeated pipettings of tubule fragments. In experiments using R26R mouse testes, testis tubule fragments containing EYFP-expressing donor cells were dissociated under UV illumination. Microinsemination was performed by intracytoplasmic injection into BDF1 oocytes. After *in vitro* culture, two-cell stage embryos were transferred into the oviducts of day 1 ICR female mice (CLEA Japan, Inc., Tokyo, Japan). Offspring were born via cesarean section on day 19.5.

### (10) Statistical analyses

Significant differences between means for single comparisons were determined by Student's t-tests. Multiple comparison analyses were carried out using ANOVA followed by Tukey's honestly significant Difference (HSD) test.

### 2. Results

### (1) Lack of germ cell transduction by adeno- or lentivirus vector

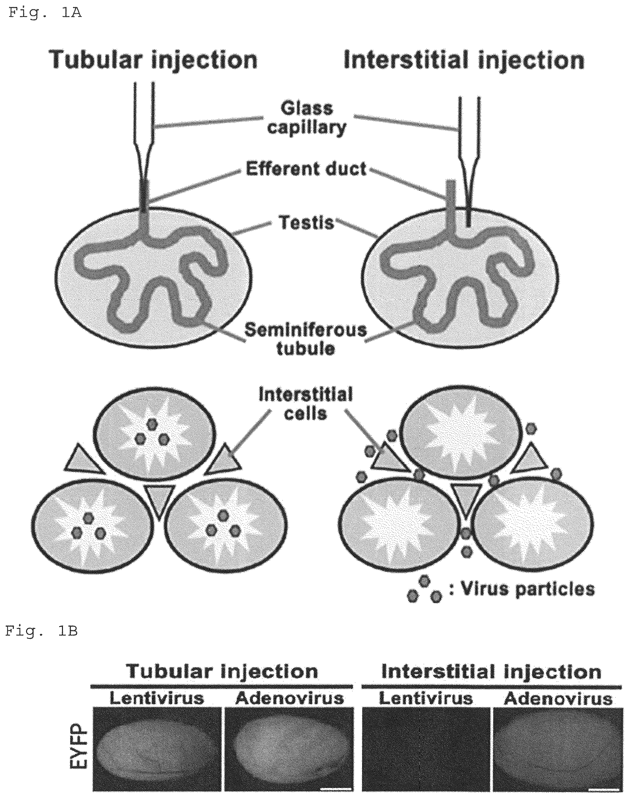

Previous studies have shown limited ability of adeno- and lentiviruses for transducing germ cells *in vivo* (Ikawa et al., 2002; Kanatsu-Shinohara et al., 2002). The infectivity of adeno- and lentivirus to germ cells was tested with R26R mice, which carry a floxed transcriptional stop element that precedes the EYFP reporter gene and express EYFP gene upon deletion of *loxP* sequences. AxCANCre or CSII-EF1-IRES-Cre was microinjected into the seminiferous tubules of R26R mice (Fig. 1A). The same virus was injected into the interstitial area as a control. When the injected testes were collected 1 week after microinjection, testes that received tubular injection showed strong EYFP fluorescence regardless of type of virus (Fig. 1B).

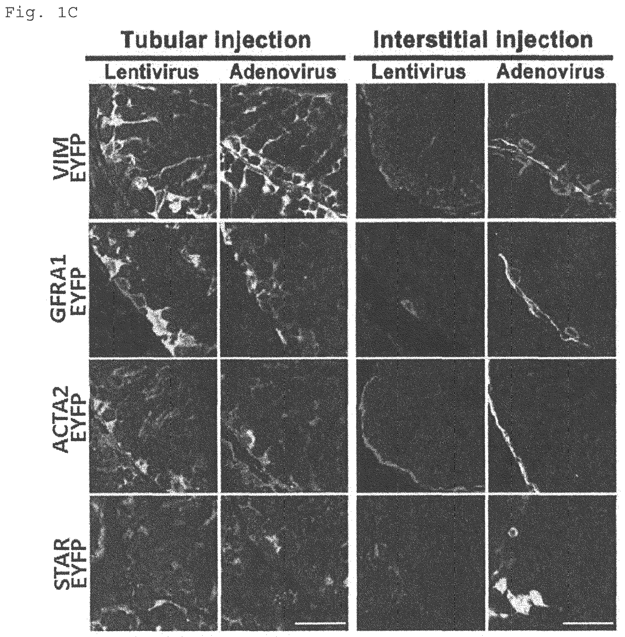

Histological sections of the testes were then prepared to examine the cell types by immunohistochemistry using markers for germ cells and Sertoli cells (GFRA1 and VIM, respectively) (Fig. 1C). Although numerous cells showed EYFP expression in the seminiferous tubules when the viruses were administered into the tubules, close examination revealed that all EYFP-expressing cells were VIM-expressing Sertoli cells. In contrast, no evidence of infection to germ cells was observed.

Interstitial injection showed different outcomes between the lentivirus and adenovirus. Although no EYFP-expressing cells was found with interstitial injection of lentivirus, relatively weak EYFP expression was observed with adenovirus. Immunostaining of histological sections revealed that both STAR-expressing Leydig cells and ACTA2-expressing peritubular cells were targeted by the adenovirus. No germ cell or Sertoli cell infection was found. Taken together, these results confirmed the previous studies that adeno- and lentivirus vectors are not useful for transducing germ cells *in vivo.*

### (2) Screening of AAV serotypes by in vivo microinjection to seminiferous tubules

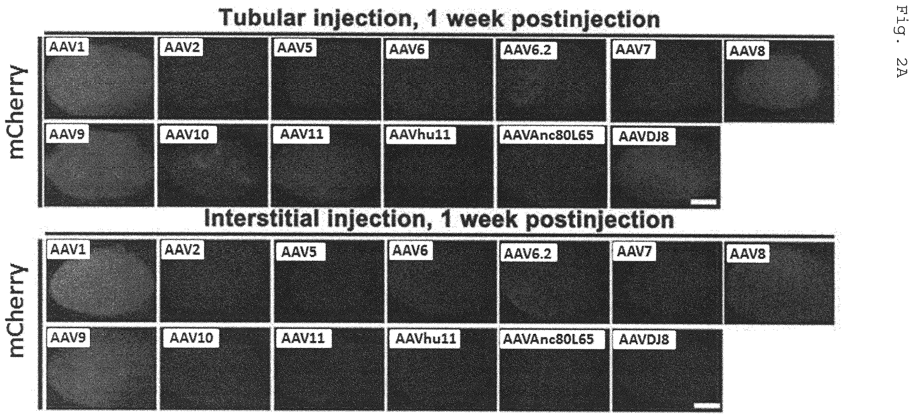

The gene transduction patterns by AAVs were then examined. Thirteen types of AAV capsids (AAV1, 2, 5, 6, 6.2, 7, 8, 9, 10, 11, hull, Anc80L65, and DJ8), all of which express mCherry under the control of the CAG promoter, were screened. Virus particles were microinjected into the seminiferous tubules or interstitial tissues. One week after infection, testes were recovered and transgene expression was examined under UV light. Of the tested 13 serotypes, analysis of transduced testes showed clear mCherry fluorescence when AAV1, 8, 9, 11, and DJ8 were microinjected into the seminiferous tubules (Fig. 2A). Focal, but consistent, infection was found for AAV6.2 and AAV10 despite repeated attempts. Relatively strong signals were observed in testes that received interstitial injection of AAV1, AAV8, and AAV9, but interstitial injection of AAVDJ8 did not show fluorescence.

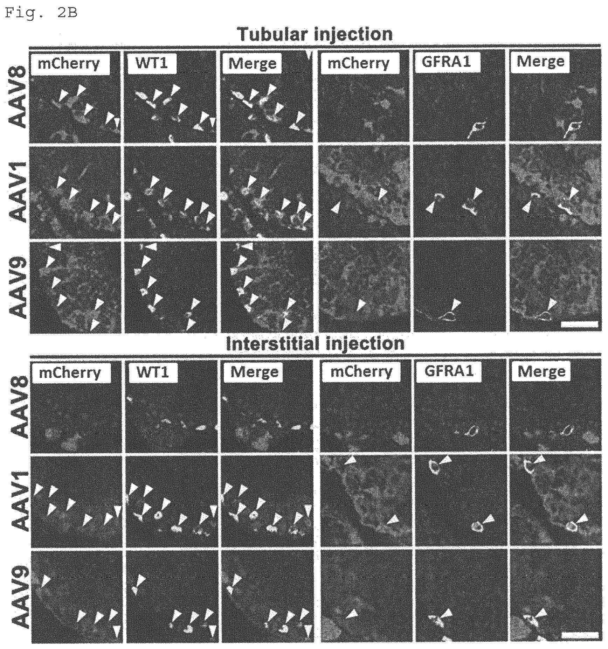

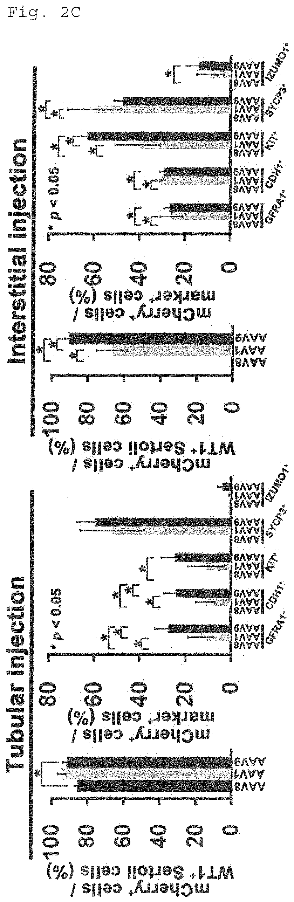

To clarify the cell type of infected cells, immunohistochemical staining was carried out. Double immunohistochemistry of testes that received tubular injection revealed that AAV8 transduced Sertoli cells selectively (Fig. 2B). No germ cell transduction was found. In contrast, AAV1 and AAV9 infected both Sertoli cells and germ cells (Fig. 2B). AAV1 and AAV9 transduced not only haploid cells in the adluminal compartment but also spermatogonia on the basement membrane. Quantification of GFRA1⁺ (A_{single} (Aₛ), A_{paired} (Aₚᵣ) spermatogonia, and some A_{aligned} (Aₐₗ) spermatogonia), and CDH1⁺ (undifferentiated spermatogonia) showed that AAV9 is superior to AAV1 in transducing these cell types (Fig. 2C). There was no significant difference in transduction efficiency for KIT⁺ (differentiating spermatogonia) or SYCP3⁺ (spermatocytes) cell stage, but IZUMO1⁺ cells (spermatids to sperms) were rarely transduced by these viruses. Very sparse signals were found in testes that received other types of AAVs. Sertoli cells were infected with comparable efficiency. These results suggest that AAV1 and AAV9 introduced into the adluminal compartment passed through the blood-testis barrier (BTB) and infected undifferentiated spermatogonia in the basal compartment.

Immunohistochemistry of the testes that received AAV1 or AAV9 interstitial injection showed very similar staining patterns to that of tubular injection. In addition to infection of STAR⁺ (Leydig) cells and ACTA2⁺ (peritubular) cells, a significant number of germ cells in both basal and adluminal compartments were infected (Fig. 2B). Signals in germ cell compartment were found beginning from GFRA1⁺ undifferentiated spermatogonia to at least IZUMO⁺ spermatid-sperm stage. Although KIT⁺ cells were constantly infected with a higher efficiency by AAV9 regardless of route of injection, GFRA1⁺ and CDH1⁺ spermatogonia were infected with comparable efficiency. Sertoli cells were more efficiently transduced by AAV9 by interstitial injection compared with AAV1. In contrast to tubular injection, no infected Sertoli cells were found by interstitial injection of AAV8.

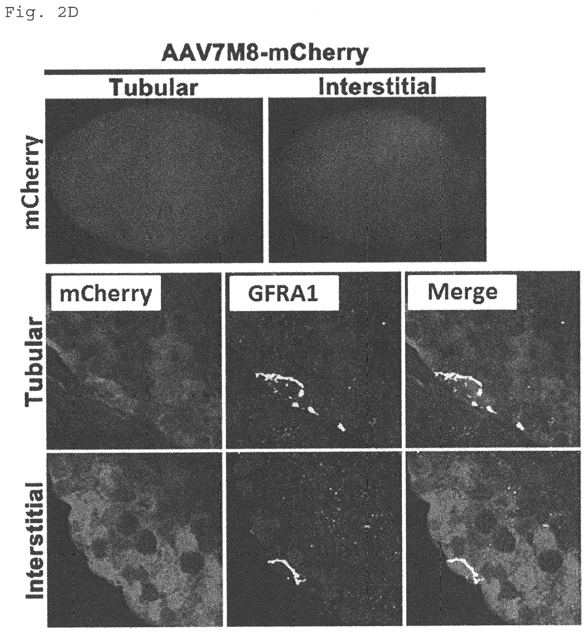

Similarly, infection of GFRA1⁺ undifferentiated spermatogonia was observed with microinjection of AAV7M8 to the seminiferous tubules or interstitium (Fig. 2D).

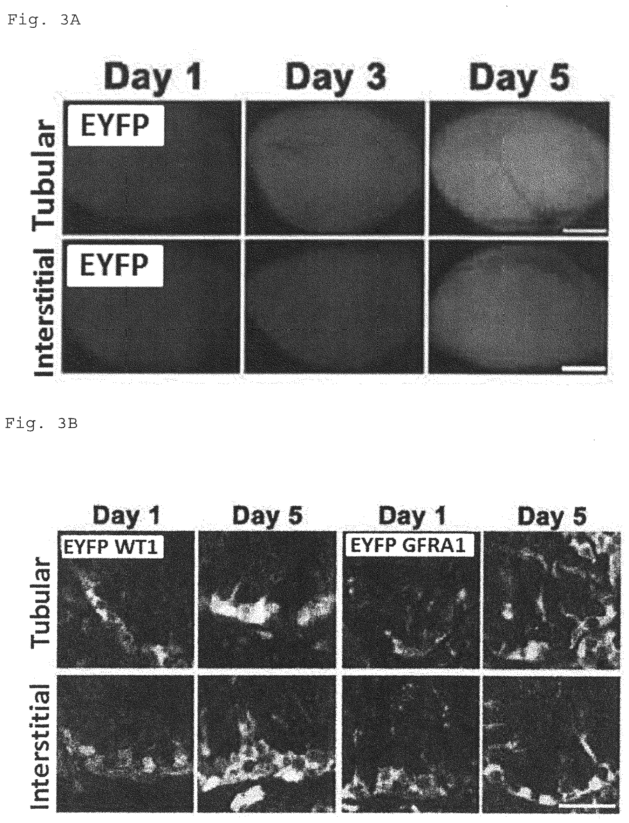

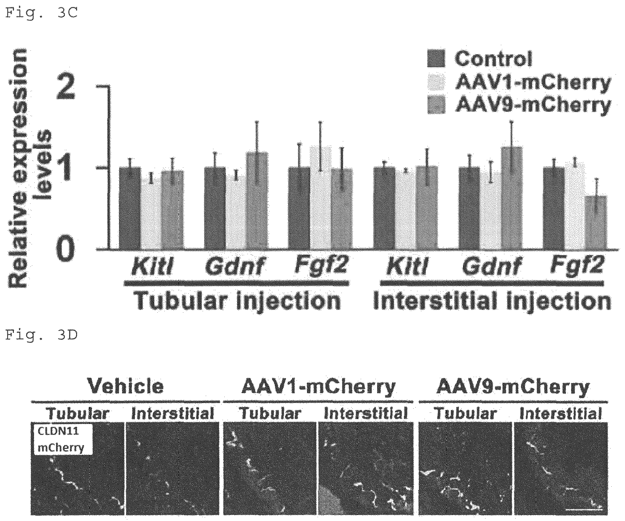

The results were confirmed with Cre-expressing AAV9 and R26R-EYFP mice. When the injected testes were compared 1, 3, and 5 days after the injection, tubular injection showed more rapid and stronger EYFP expression (Fig. 3A). Immunostaining showed that WT1⁺ Sertoli cells were infected as early as 1 day after microinjection when GFRA1⁺ spermatogonia did not show fluorescence (Fig. 3B), suggesting that Sertoli cell infection is a very rapid process. The infection of AAV did not appear to influence the SSC microenvironment significantly because real-time PCR showed that neither AAV1 nor AAV9 transduction influenced the expression levels of cytokines involved in spermatogonia self-renewal and differentiation (Fig. 3C). Moreover, immunostaining showed that CLDN11, a major component of the BTB, did not show apparent changes by AAV1 and AAV9 infection (Fig. 3D).

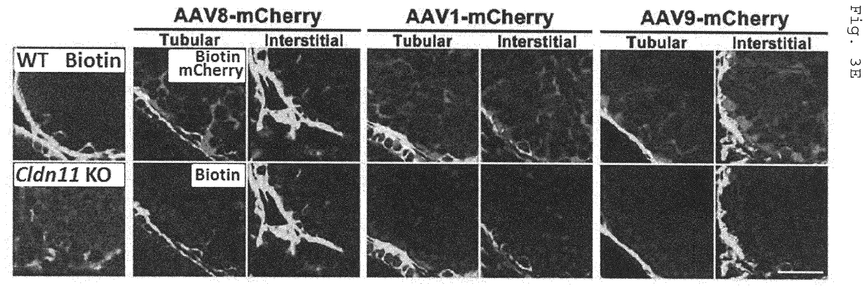

Whether AAV microinjection disturbs the BTB integrity was tested with tracer experiments. Three days after tubular or interstitial injection of AAV1- or AAV9-mCherry, biotin (557 D) was microinjected into the interstitium of adult testes. *Cldn11* knockout mouse testes, which do not have the BTB, were used as a positive control. Although leakage into the tubule lumen was observed in *Cldn11* KO mice (Fig. 3E), AAV injection did not induce apparent biotin leakage into the adluminal compartment, which suggested that AAV infection does not compromise the integrity of the BTB. Because GFRA1 is thought to be expressed in SSCs, this result suggested that both AAV1 and AAV9 passed through the basement membrane as well as the BTB and infected both Sertoli cells and germ cells, possibly including spermatogonial stem cells (SSCs).

### (3) In vivo transduction of SSCs by AAV1/9

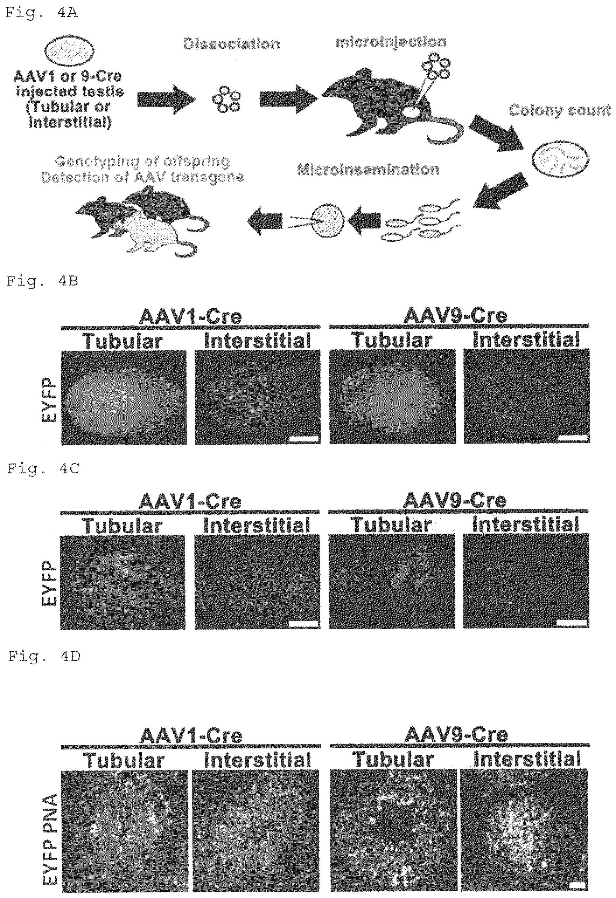

To confirm the transduction of SSCs, spermatogonial transplantation was carried out (Brinster and Zimmermann, 1994) (Fig. 4A). In this experiment, Cre-expressing AAV1 or AAV9 (AAV1-Cre or AAV9-Cre) was microinjected into the testes of R26R-EYFP mice by tubular or interstitial injection. Between 1 and 2 weeks after microinjection, the mice were sacrificed and their testes were recovered. Testes of R26R-EYFP mice that received tubular injection showed strong fluorescence under the UV light (Fig. 4B). In contrast, EYFP expression was not apparent in testes that received interstitial injection at this point. Donor testes were dissociated into single cells by enzymatic digestion, and were microinjected into the seminiferous tubules of busulfan-treated mice, which lack endogenous spermatogenesis.

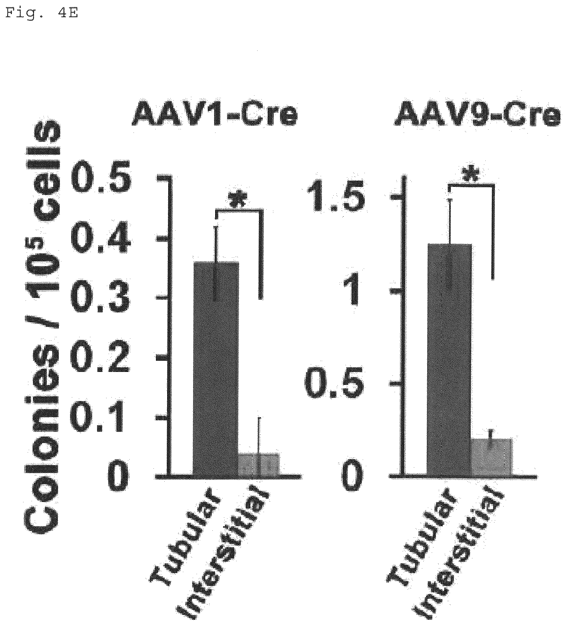

When recipient testes were analyzed 2 months after transplantation, all donor types produced germ cell colonies in recipient testes, which demonstrated that SSCs were transduced by AAV1/9 regardless of route of injection (Fig. 4C). Histological analyses showed normal appearing spermatogenesis in the recipient testes regardless of donor cell type and route of injection (Fig. 4D). However, quantification of colonies showed that the frequency of colonization depends on the type of virus and route of injection. R26R testis cells that received AAV1 and AAV9 via tubular injection produced 0.4 and 1.2 colonies per 10⁵ transplanted cells, respectively (Fig. 4E). On the other hand, the numbers of colonies generated by AAV1 and AAV9 interstitial injection were 0.04 and 0.21 per 10⁵ cells, respectively. In both cases, significantly smaller numbers of colonies were found when R26R testes with interstitial injection were used as donors.

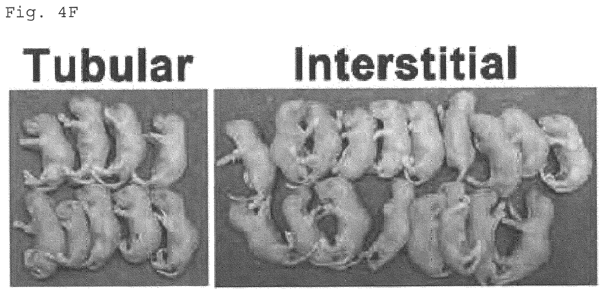

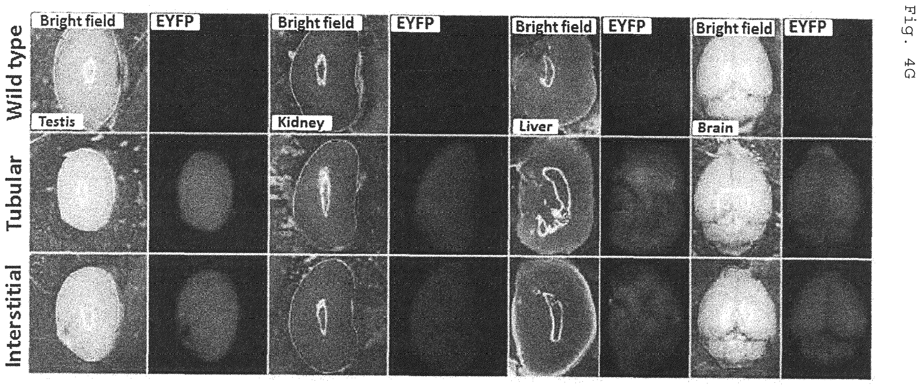

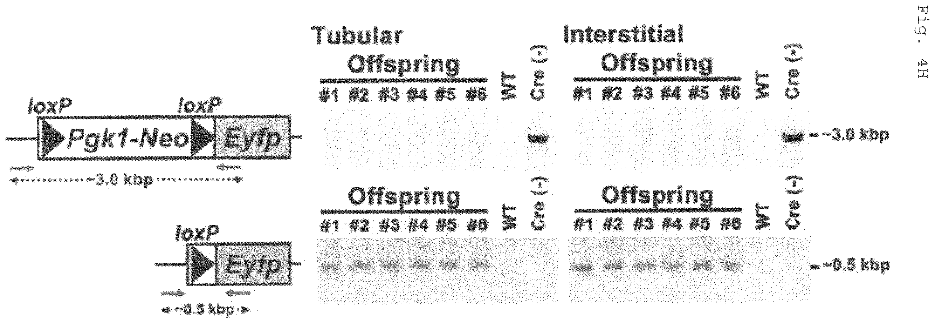

To confirm the function of Cre-transfected SSCs, *in vitro* microinsemination was carried out. Elongated spermatids were collected from recipient mice to which germ cells from testes that received tubular or interstitial injection of AAV9 were transplanted, and microinjected into oocytes (Table 1). Both types of cells produced normal offspring and a total of 10 and 18 offspring were born, respectively (Fig. 4F). The offspring showed EYFP fluorescence under UV light (Fig. 4G), and deletion of *loxP* was confirmed (Fig. 4H). These results showed that SSCs that were transduced by AAV9 *in vivo* are functionally normal regardless of route of infection.

**Table 1**

| Development of oocytes fertilized with spermatids recovered from AAV9-transduced SSCs | | | | |
|---|---|---|---|---|
| Type of experiment | No. of embryos cultured | No. of embryos transduced (%) | No. of embryos implanted (%) | No. of pups (%) |
| Tubular | 57 | 30 (73.7) | 12 (21.0) | 10 (17.5) |
| Interstitial | 58 | 43 (74.1) | 26 (44.8) | 18 (31.0) |

### (4) Improvement of transfection of AAV9 by neuraminidase

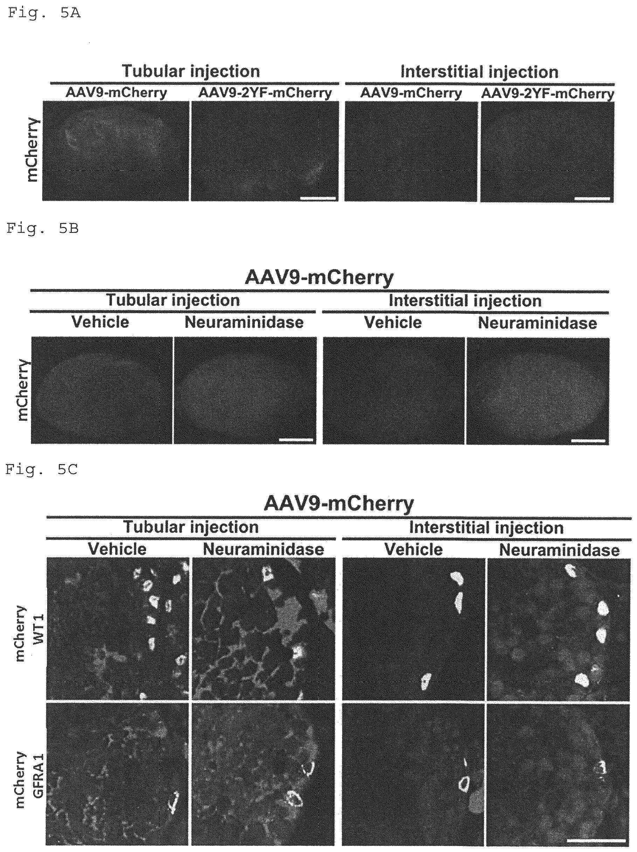

To improve the efficiency of AAV-mediated gene transduction, an AAV9 mutant capsid was used. It has been shown that tyrosines exposed on the AAV9 capsid surface can undergo tyrosine kinase-mediated phosphorylation, which leads to ubiquitination and degradation of viral particles. Site-directed tyrosine to phenylalanine mutagenesis of two of the seven capsid residues at positions 446 and 731 (AAV9-2YF) is known to delay ubiquitination and have been used to improve the transfection efficiency. However, apparent improvement was not observed with AAV9-2YF (Fig. 5A).

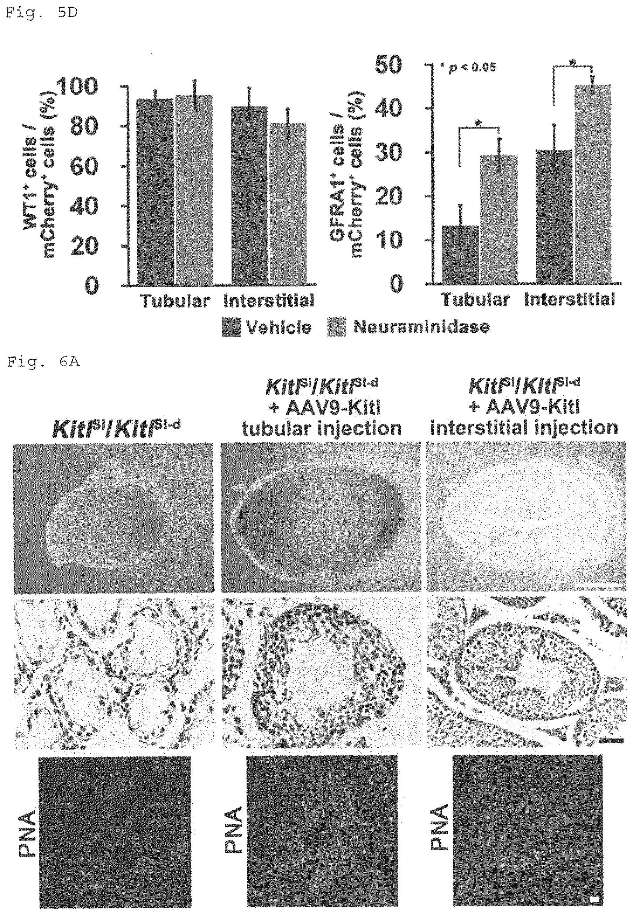

Neuraminidase was then used to improve AAV infection efficiency. Attachment to cell surface glycans is the critical step in the AAV infectious pathway, and AAV9 uptake in lungs was greatly increased when terminal sialic acids were removed by neuraminidase. AAV9-mCherry was co-injected with neuraminidase into the seminiferous tubules as well as interstitial tissue of wild-type mice. When the mice were sacrificed 1 week after the injection, testes that received co-injection of neuraminidase showed significantly enhanced mCherry expression regardless of route of injection (Fig. 5B). Double immunostaining and quantification of the tubule counts showed that neuraminidase significantly increased transduction efficiency of GFRA1⁺ undifferentiated spermatogonia by approximately 2.2 and 1.5 times in tubular and interstitial injections, respectively (Figs. 5C and 5D). No significant differences in Sertoli cell transduction was observed, possibly because these cells were already infected with high efficiency even without any treatment. These results showed that neuraminidase treatment can improve AAV9 transduction efficiency in undifferentiated spermatogonia.

### (5) Rescue of infertility in Kitl^{sl}/Kitl^{sl-d} mice by AAV9 transduction of Sertoli cells

The utility and safety of AAV were examined by correcting infertility of *Kitl^{sl}*/*Kitl*^{*sl*-*d*} male mice. These mice are congenitally infertile because they lack expression of membrane-bound *Kitl* in Sertoli cells. However, these testes contain a small number of SSCs that do not depend on membrane-bound KITL for survival (estimated to be 5% or less of wild-type number) . Testes of *Kitl^{sl}*/*Kitl*^{*sl*-*d*} mice are smaller than those of wild-type mice and no apparent germ cells are found by histological analysis (Fig. 6A). AAV9-Kitl was microinjected into the seminiferous tubules as well as into the interstitial tissue. Neuraminidase was also added in some experiments to improve transduction efficiency.

The mice were sacrificed three months after microinjection to examine the levels of spermatogenesis. While the *Kitl^{sl}*/*Kitl*^{*sl*-*d*} mouse testis was small (approximately 11 mg), the testis that received AAV9-Kitl injection grew larger regardless of route of injection (Fig. 6A), which suggested regeneration of spermatogenesis. Histological analyses of the injected testes revealed spermatogenesis in all of the samples. The success of the restoration of spermatogenesis varied greatly among the samples, but comparable numbers of seminiferous tubules exhibited spermatogenesis by either type of injection. In the most successful case, approximately 77.1% and 64.1% of the seminiferous tubules contained spermatogenesis by intratubular and interstitial microinjection, respectively (Table 2). Unlike adenovirus-mediated gene transduction, no apparent inflammatory reaction was found by histological analysis (Fig. 6A). These results suggested that Sertoli cells can be transduced by AAV vectors for restoration of spermatogenesis regardless of administration route.

**Table 2. Spermatogenesis following microinjection of AAV9-Kitl into Kitl^{sl}/Kitl^{sl-d} mouse testes**

| Type of experiment | Sample number | Virus titer (viral particles/ml) | Days after injection | Testis weight^{a} | Tubule with spermatogenesis (%)^{b} |
|---|---|---|---|---|---|
| Tubular | 1 | 1.0 × 10¹¹ | 92 | 21.5 | 43.2 (48/111) |
| | 2 | 1.0 × 10¹¹ | 92 | 29.8 | 77.1 (91/118) |
| | 3 | 1.0 × 10¹¹ | 92 | 22.1 | 28.7 (56/195) |
| | 4 | 5.0 × 10¹² | 126 | 30.0 | 33.9 (43/127) |
| | 5 | 5.0 × 10¹² | 126 | 36.8 | 50.4 (67/133) |
| Interstitial | 1 | 5.0 × 10¹² | 91 | 21.7 | 21.5 (31/144) |
| | 2 | 5.0 × 10¹² | 91 | 28.5 | 64.1 (66/103) |
| | 3^{c} | 5.0 × 10¹² | 91 | 27.6 | 53.8 (64/119) |
| | 4^{c} | 5.0 × 10¹² | 91 | 24.0 | 24.0 (42/175) |
| | 5^{c} | 5.0 × 10¹² | 94 | 23.5 | 38.9 (44/113) |

| | | | | | |
|---|---|---|---|---|---|
| ^{a}Average weight of untreated *Kitl*^{sl}/*Kitl*^{sl-d} mouse testes was 11.0 mg (n = 4). ^{b}In parenthesis, total number of tubule cross-sections containing spermatogenesis/total number of cross-sections examined is indicated. ^{c}Neuraminidase was co-injected with AAV9-Kitl. | | | | | |

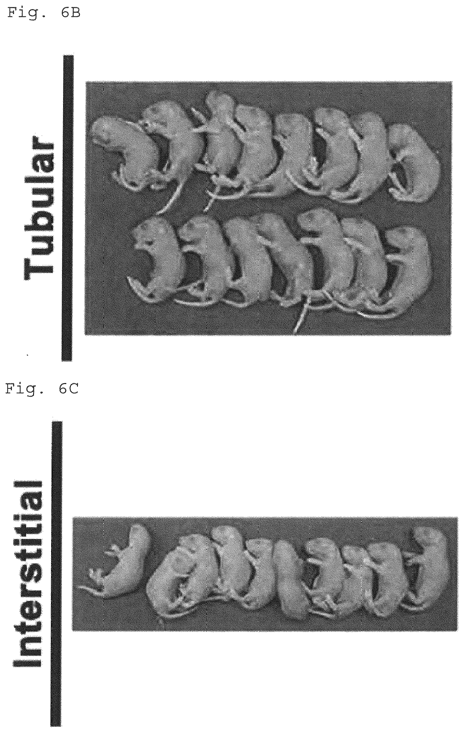

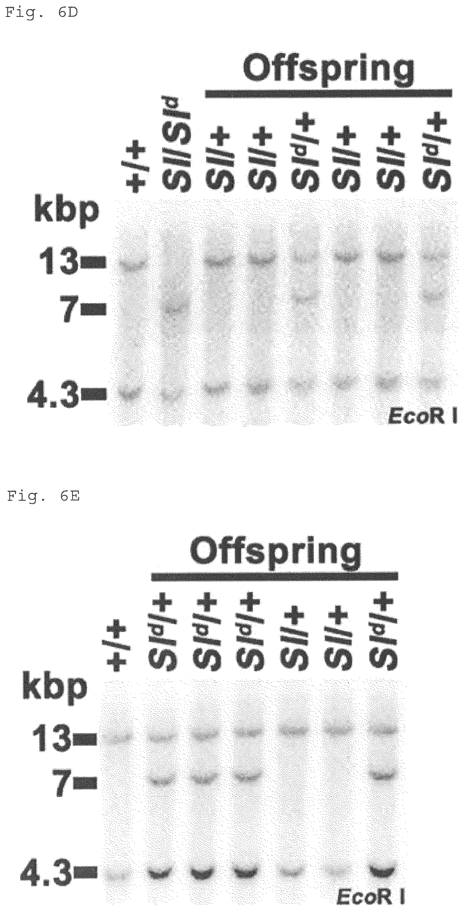

To obtain offspring from the *Kitl^{sl}*/*Kitl*^{*sl*-*d*} mice, microinsemination was carried out. Testes from both types of mice were refrigerated overnight and were used for microinsemination on the next day. Seminiferous tubules were dissociated by repeated pipetting, and round or elongated spermatids were used for microinjection into oocytes. Using sperm from *Kitl^{sl}*/*Kitl*^{*sl*-*d*} testes that received tubular or interstitial injection of AAV9-Kitl, a total of 135 and 118 embryos were produced and 98 (72.6%) and 100 (84.7%) of these embryos developed into 2-cell stage embryos 48 hours after microinsemination, respectively. All of these 2-cell stage embryos were transferred into the uteri of pseudopregnant mothers. Thirty-one and 27 offspring were born, respectively, from *Kitl^{sl}*/*Kitl*^{*sl*-*d*} mice that had received tubular and interstitial injections of AAV9-*Kitl* (Figs. 6B and 6C) . Southern blot analysis showed that each offspring carried an *Kitl*^{sl} or *Kitl*^{sl-d} mutant allele (Figs. 6D and 6E) .

### (6) Lack of germline AAV9 integration

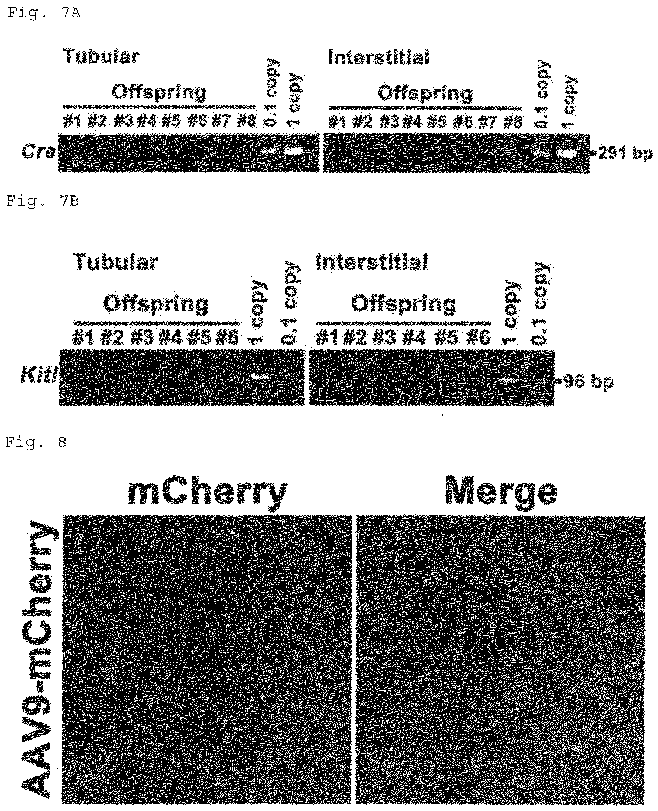

Since the SSC and Sertoli cell transductions showed the feasibility of AAV9 to infect these cell types, the offspring produced by these methods may contain AAV9 genome. To check this possibility, PCR analysis was carried out using tail DNA collected from all offspring born from SSC or Sertoli cell transduction experiments. Analyses revealed that none of the offspring contained AAV9 DNA (Figs. 7A and 7B). These results indicate that AAV9 can transduce both SSCs and Sertoli cells without integration into the genome.

### Example 2

AAV infection to rabbit testes was examined by transplanting rabbit testicular fragments to mouse testes. First, busulfan (44 mg/kg) was administered intraperitoneally to male nude mice (KSN/nu) to prepare nude mice with the testis being deficient in intrinsic spermatogenesis. The testes that lost spermatogenesis due to this process were small while maintaining environments required for growth and maintenance of sperm stem cells and progress of spermatogenesis and thus suitable for transplantation of testis fragments. Next, the testis was removed from a 16-week old New Zealand White rabbit to prepare testicular fragments of approximately 2 mm square with scissors. The testis fragments were transplanted into the testes of busulfan-treated nude mice. After one week from the transplantation, 10 µl of AAV9-CAG-mCherry (1.0 × 10¹⁴ viral particles/ml) was injected into the testis interstitium. The testes were harvested at 1 week after injection, and tissue sections were prepared and immunostained with an anti-mCherry antibody. Round cell shape suggested infection to germ cells, and it was shown that AAV9 injected into the interstitium infected cells in the seminiferous tubules of rabbits (Fig. 8).

### Example 3

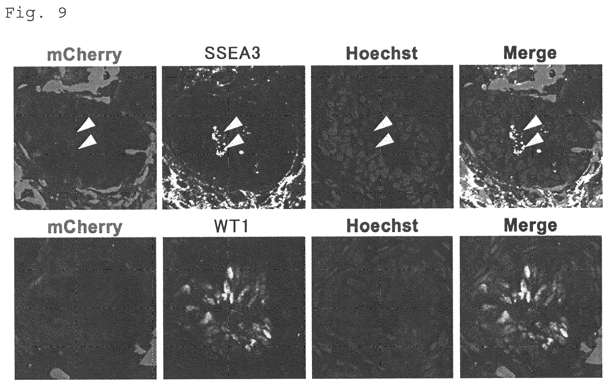

In the same manner as in Example 2, marmoset testicular fragments were transplanted into the testes of the nude mice. The testis was removed from a marmoset (Callithrix jacchus), and testicular fragments of approximately 2 mm square was prepared with scissors and transplanted into the testes of busulfan-treated nude mice. After one week from the transplantation, 10 µl of AAV9-CAG-mCherry (1.0 × 10¹⁴ viral particles/ml) was injected into the testis interstitium. The testes were harvested at 1 week after injection, and tissue sections were prepared and immunostained with anti-mCherry, anti-SSEA3, and anti-WTl antibodies. Expression of mCherry was confirmed in cells expressing a spermatogonia marker SSEA3 and those expressing a Sertoli cell marker WT1. It was shown that AAV9 injected into the interstitium infected with spermatogonia and Sertoli cells in the seminiferous tubules of marmosets (Fig. 9).

## Claims

1. A composition comprising an adeno-associated virus vector for introducing a polynucleotide into a male germ cell or a Sertoli cell in the testis of a vertebrate.

2. The composition according to claim 1, wherein the composition is for treating male infertility.

3. The composition according to claim 1 or 2, wherein the vertebrate is a human.

4. The composition according to any one of claims 1-3, wherein the adeno-associated virus vector is AAV1, AAV9 or AAV7M8.

5. A method of introducing a polynucleotide into a male germ cell or a Sertoli cell, comprising injecting an adeno-associated virus vector comprising the polynucleotide into the testis of a non-human vertebrate.

6. A method of producing a non-human vertebrate comprising a male germ cell or a Sertoli cell into which a polynucleotide is introduced, comprising injecting an adeno-associated virus vector comprising the polynucleotide into the testis of a non-human vertebrate.

7. The method according to claim 5 or 6, wherein the adeno-associated virus vector is AAV1, AAV9 or AAV7M8.

8. The method according to any one of claims 5-7, wherein the male germ cell is a spermatogonial stem cell.

9. The method according to any one of claims 5-8, wherein the vertebrate has the blood-testis barrier.

10. The method according to any one of claims 5-9, wherein the adeno-associated virus vector is injected into the testis interstitium.

11. A method of producing a genetically modified non-human vertebrate, comprising
injecting an adeno-associated virus vector into the testis of a non-human vertebrate to form a genetically modified sperm, and
fertilizing an egg with the genetically modified sperm to obtain a genetically modified individual.

12. A method of producing a genetically modified sperm, comprising injecting an adeno-associated virus vector into the testis of a non-human vertebrate to form a genetically modified sperm.

13. The method according to claim 11 or 12, wherein the adeno-associated virus vector is AAV1, AAV9 or AAV7M8.

14. The method according to any one of claims 11-13, wherein the vertebrate has the blood-testis barrier.

15. The method according to any one of claims 11-14, wherein the adeno-associated virus vector is injected into the testis interstitium.
